# EUROPEAN PATENT APPLICATION

(11) **EP 3 028 742 A1**
(43) Date of publication of application: **08.06.2016**
(21) Application number: 14196590.5
(22) Date of filing: 05.12.2014
(51) Int. Cl.: A61N 2/00, A61N 2/06, A61N 5/06

(54) **Handheld combined magnetic and light therapy device**

(71) Applicant: Mikropis Holding d.o.o. Zalec, 3310 Zalec (SI)
(72) Inventor: Uplaznik, Janez, 3310 Zalec (SI); Laptev, Boris, 1000 Ljubljana (SI); Sidorenko, Galina, 1000 Ljubljana (SI)
(74) Representative: Lucke, Andreas

(57) **Abstract**

Disclosed herein is a handheld combined magnetic and light therapy device (10), comprising a treatment surface (18) that is to be positioned adjacent to the body part to be treated during operation; a two-dimensional array (42) of at least four anti-parallel magnets (32) and one or more light sources (26, 28, 30) arranged to emit visible and/or IR light from said treatment surface (18).

## Description

### FIELD OF THE INVENTION

The present invention is in the field of medical and therapeutic equipment. In particular, the invention relates to a hand-held combined magnetic and light therapy device.

### BACKGROUND OF THE INVENTION

In prior medical technologies, the therapeutic effect of both, light and magnetic fields for therapeutic purposes has been extensively exploited. In the context of the present disclosure, the term "light" shall refer to electro-magnetic radiation both in the visible spectrum as well as in the near-infrared spectrum, i.e. with wavelengths up to 1000 nm. Further, in the present invention, magnetic therapy shall refer to an application of static or at least slowly varying magnetic fields. The term "magnetic therapy" shall not refer to the magnetic field component of electromagnetic waves such as microwaves, light etc.

A combined light therapy and magnetic therapy device is known from US 2013/0211180. The device includes a shell designed to be gripped by a user, including an outer surface shaped by at least three flat areas, and an inner bore-hole formed inside the shell for receiving first and second magnets in close connection with each other. Both magnets have north and south pole surfaces and an axis, respectively, aligned with each other, and the north pole surface of the first magnet is exposed on a surface of a shell. Windows having an inner rim are arranged on each flat area of the shell. Said close connection is established between the south pole surface of the first magnet and north pole surface of the second magnet. At least one infrared radiating tablet is fixed in said window and abutted against said rim by a clamp placed inside said bore-hole.

In US 2014/0100410 A1, a multi-layer magnetic device is disclosed which comprises two or more layers of permanent magnets of ferromagnetic material to be applied to areas of the body of a mammal. The first layer has one or more protrusions and the second layer has one or more receiving zones that are positioned to align with the protrusions of the first layer. At least a portion of the first layer has a first magnetic pattern, and at least a portion of the second layer has a second magnetic pattern such that, when the protrusions are aligned with the receiving zones, at least one region of the first magnetic pattern faces at least one region of the second magnetic pattern of light polarity, thereby repulsing one another. The magnetic configuration can for instance be placed in insoles of shoe. As the user applies his or her weight to the insole, the weight forces the layers together, closing an air gap created by the magnetic repulse between the two layers and thereby leading to a "pumping" of magnetic flux.

In Russian Patent No. 2134601, "Device for magnetic therapy and light therapy" by *Hristoforov V.N., Hristoforova T.V. and Grabovshchiner A.J.,* a combined therapeutic and magnetic device is disclosed which comprises permanent magnets, a switching unit for switching on and off LEDs and a modulator for modulating the amplitude of the light sources.

### SUMMARY OF THE INVENTION

The treatment provided by the prior art magnetic therapy devices or combined magnetic and light therapy devices is not ideal in all respects. In particular, there is an ongoing need to increase the efficiency of the magnetic therapy.

The problem underlying the invention is therefore to improve a hand-held combined magnetic and light therapy device particularly with respect to the efficiency of the magnetic therapy.

This problem is solved by a hand-held combined magnetic and light therapy device according to claim 1. Preferable embodiments are defined in the dependent claims.

According to the invention, the handheld combined magnetic and light therapy device comprises a treatment surface that is to be positioned adjacent to the body part to be treated, a two-dimensional array of at least four anti-parallel magnets, and one or more light sources arranged to emit visible and/or IR-light from said treatment surface.

According to the invention, when the treatment surface of the device is positioned adjacent to the body part to be treated, the body part can simultaneously be exposed to the magnetic field of the two-dimensional array of at least four anti-parallel magnets, and can simultaneously be irradiated with visible and/or IR-light. The array of at least four anti-parallel magnets leads to significant gradients in the magnetic field strength. Also, when the array is moved relative to the treated body part, the magnetic field experienced by the tissue changes abruptly which has been observed to give rise to a significant therapeutic effect.

Note that the term "anti-parallel" as used in the present disclosure shall not imply that the magnets must have truly parallel axes, but is rather meant to define that the respective dipole moments at least have components that are directed in opposite directions, or in other words, that the north pole of one magnet is closer to the south pole of the adjacent magnet than to the adjacent magnet's north pole. However, preferably the magnets are precisely anti-parallel in the sense that the dipole moments are precisely opposite.

Preferably, the distance between adjacent anti-parallel magnets is less than 1 mm, more preferably less than 0.1 mm, and most preferably less than 0.05 mm.

With such small gaps between the anti-parallel magnets of the array, the changes in magnetic field experienced by the tissue become even more abrupt, and the therapeutic effect is considerably enhanced. Further, employing a two-dimensional array of at least four anti-parallel ensures that the change in magnetic field occurs irrespectively of the direction in which the device is moved on the surface of the body part to be treated, which further increases the therapeutic effect.

Preferably, the north-south-axis of the magnets is perpendicular to the treatment surface or deviates from perpendicular by 15 ° or less.

In a preferred embodiment, the two-dimensional array of anti-parallel magnets is a rectangular array comprised of perpendicular rows and columns.

In a preferred embodiment, the two-dimensional array of anti-parallel magnets is provided on a moveable magnet carrier, on which in addition to the array of anti-parallel magnets, one or more of
- a single permanent magnet having a north pole facing the treatment surface when in operating position,
- a single magnet having a south pole facing the treatment surface when in operating position and
- a magnetic screen
is or are provided.

This way, instead of the alternating magnetic field provided by the array of anti-parallel magnets, the treatment can also selectively be carried out only with a north pole pointing to the treated array or only with the south pole pointing to the treated are or without any magnetic field.

In a preferred embodiment that will be shown in detail below, the moveable magnet carrier can be rotatable, for example rotatable around an axis that is parallel to the treatment surface.

In a preferred embodiment, the one or more light sources comprises
- one or more red light sources having a peak wavelength in the range of 600 nm to 790 nm,
- one or more blue light sources having a peak wavelength in the range of 450 nm to 480 nm, and
- one or more IR light sources having a peak wavelength in the range of 790 nm to 1000 nm.

The specific therapeutic effects of the different light sources, particularly in combination with the magnetic field, will become more apparent from specific treatment procedures described below.

Preferably, the light sources are LEDs, since LEDs are cheap, reliable and have a high power efficiency.

Preferably, the light sources are evenly distributed, so that the body part to be treated can likewise be evenly irradiated. In a particularly preferred embodiment, the light sources are distributed circumferentially around the two-dimensional array of antiparallel magnets.

In a preferred embodiment, the device further comprises a plurality of protrusions provided on top of the treatment surface, said protrusions being suitable for use in a massage when the treatment surface is lightly pressed against and moved relative to the treated body part.

It has been found that the combination of magnetic therapy, light therapy and massage when carried out simultaneously has a beneficial therapeutic effect, for example for the general improvement of health and stimulation of the immune system or in a treatment accompanying fitness training, to name but two examples.

In a preferred embodiment, the device comprises a transparent cover. The transparent cover can collectively cover all of the light sources, which then can be easily mounted on a suitable carrier without needing individual covers of their own. The transparent cover can also be easily cleaned after use.

In a particularly preferred embodiment, the protrusions are formed on the transparent cover.

In a preferred embodiment, the device further comprises a battery for powering the light sources. The use of a battery allows for a convenient portable device that can be conveniently used.

In a preferred embodiment, the device further comprises a modulating unit for modulating the intensity of one or more of the light sources with at least one frequency between 0.05 Hz and 20 kHz. It has been observed that the effect of the light therapy can be in some applications be improved if the amplitude of the light intensity is modulated at such frequencies.

In a preferred embodiment, the modulation unit is configured to carry out modulations at at least three different modulation frequencies selected from an interval of 0.1 Hz to 10 kHz.

In a preferred embodiment, the device further comprises a switching unit configured to selectively switch on one or more of
- an individual light source (26, 28, 30)
- a group of plural or all of the light sources (26, 28, 30) having the same color,
- two or more groups of light sources (26, 28, 30) having the same color.

Finally, the device may have a housing having a handle portion to be held by a user and a treatment portion on which the treatment surface is provided.

### SHORT DESCRIPTION OF THE FIGURES

- Fig. 1a and 1b: show front and sectional side views of handheld combined magnetic and radiation therapy device according to an embodiment of the present invention.
- Fig. 2a and 2b: show front and sectional side views of handheld combined magnetic and radiation therapy device according to an alternative embodiment of the present invention.
- Fig. 3: is a block diagram showing the electrical components of the device of Figs. 1 and 2.

### DESCRIPTION OF THE PREFERRED EMBODIMENT

For the purposes of promoting an understanding of the principles of the invention, reference will now be made to the preferred embodiments illustrated in the drawings and specific language will be used to describe the same. It will nevertheless be understood that no limitation of the scope of the invention is thereby intended, such alterations and further modifications in the illustrated device and method and such further applications of the principles of the invention as illustrated therein being contemplated therein as would normally occur now or in the future to one skilled in the art to which the invention relates.

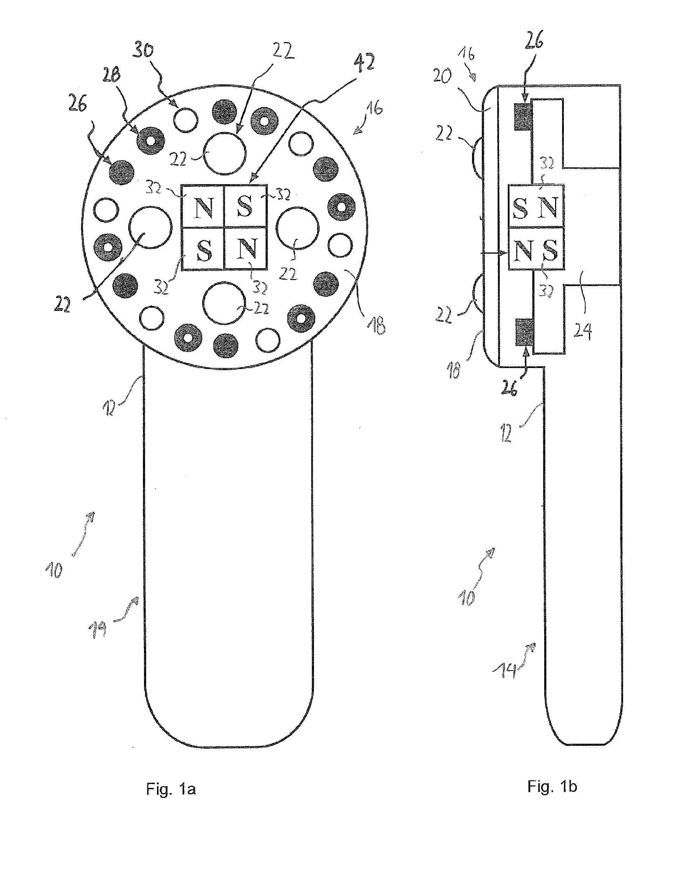

Fig. 1a shows a front view and Fig. 1b a sectional side view of a handheld combined magnetic and radiation therapy device 10 according to an embodiment of the present invention. The handheld device 10 comprises a housing 12 having a handle portion 14 and a treatment portion 16. On the treatment portion 16, a treatment surface 18 is found which is a surface that is to be placed adjacent to a surface of a treated body part under operation. In the shown embodiment, the treatment surface 18 is formed by a transparent cover 20, on which four protrusions 22 are formed. In the present example, the shape of the protrusions 22 is approximately hemispherical, but other shapes are possible as well. Also, the number of protrusions is not limited to four. As will become apparent from the detailed description below, the purpose of the protrusions 22 is to provide a massage function upon operation of the device 10.

Inside the treatment portion 16 of the housing 12, a carrier 24 is provided on which three types of light sources 26, 28, and 30 are evenly circumferentially arranged. In the present example, each of the light sources 26, 28 and 30 are formed by LEDs. The first type of light source 26 is configured to emit red light having a peak wavelength in the range of 600 to 790 nm. The red light sources 26 are represented as closed circles in Fig. 1.

The second type of light source 28 is formed by blue light sources having a peak wavelength in the range of 450 to 480 nm. The blue light sources 28 are represented by a closed circle with a small white circle in the center.

Finally, the third type of light source 30 is an IR light source, having a peak wavelength in the range of 790 to 1000 nm.

In the preferred embodiment, the power densities of each of the light sources 26, 28, 30 is between 2 and 10 mW/cm², preferably between 5 and 7 mW/cm².

Further on the carrier 24, a two-dimensional array 42 of four permanent magnets 32 is provided. Herein, the term "two-dimensional array" shall mean that at least one of the magnets 32 has directly adjacent neighboring magnets in at least two non-parallel directions. In the presently preferred embodiment, the two-dimensional array has a rectangular grid structure, but the invention is not limited to this. Further, the magnetic dipole moment of each of the magnets 32 is perpendicular to the treatment surface 18.

The anti-parallel orientation of the permanent magnets 32 is such that some of the magnets have their north poles facing the treatment surface 18, while their direct neighbors have their south poles facing the treatment surface 18.

The spacing between adjacent permanent magnets 32 is very small. In the preferred embodiments, any clearance or gap between adjacent anti-parallel magnets 32 is less than 1 mm, preferably less than 0.1 mm and most preferably 0.05 mm or less.

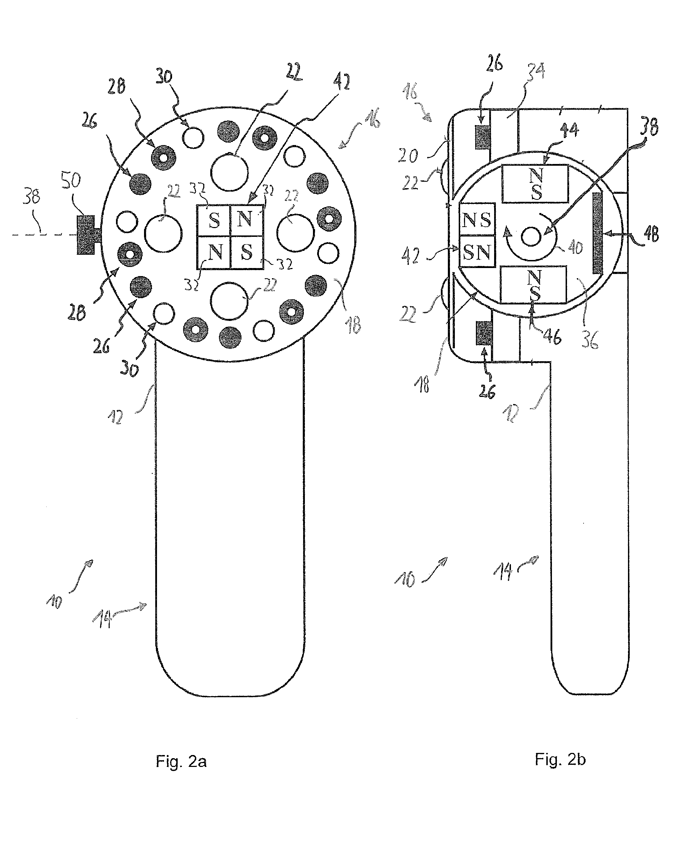

Fig. 2a and Fig. 2b show front and sectional side views of a further embodiment of the device 10 according to the present invention. Similar or corresponding components of the device 10 are denoted with the same reference signs as in Fig. 1, and the description is not repeated. The main difference of the device 10 of Fig. 2 as compared to that of Fig. 1 is that instead of a single carrier 24 for carrying both the light sources 26, 28 and 30 as well as the permanent magnets 32, the device 10 of Fig. 2 comprises a carrier 34 for the light sources 26, 28 and 30 and an additional magnet carrier 36 that is rotatable around an axis 38, as indicated by the arrow 40 in Fig. 2b.

On the rotatable magnet carrier 36,
- an array 42 of anti-parallel permanent magnets 32 (similar to the embodiment of Fig. 1)
- a single permanent magnet 44 with its north pole facing the treatment surface 18 when in operating position,
- a single magnet 46 with its south pole facing the treatment surface 18 when in operation position, and
- a magnetic screen 48 are provided. Instead of the single permanent magnets 44, 46, an array of individual permanent magnets oriented in parallel can be used.

By turning an operating handle 50 (see Fig. 2a), which in the present embodiment is a simple knob, each of the array 42, single magnets 44 and 46 or the magnetic screen 48 can be selectively positioned to be adjacent to the treatment surface 18.

In the position shown in Fig. 2b, the array 42 of anti-parallel permanent magnets faces the treatment surface 18, such that the functionality of the device is similar to that shown in Fig. 1. However, by turning the magnetic carrier 36 appropriately, one of the single permanent magnets 44 and 46 can be positioned tube adjacent to the treatment surface 18, thereby allowing for a treatment with a magnetic field of a single, selectable polarity (north or south pole). Finally, when the magnetic carrier 36 is rotated such that the magnetic screen 48 faces the treatment surface 18, no or very little magnetic flux will penetrate the treatment surface 18, such that the magnetic modality is essentially "switched off".

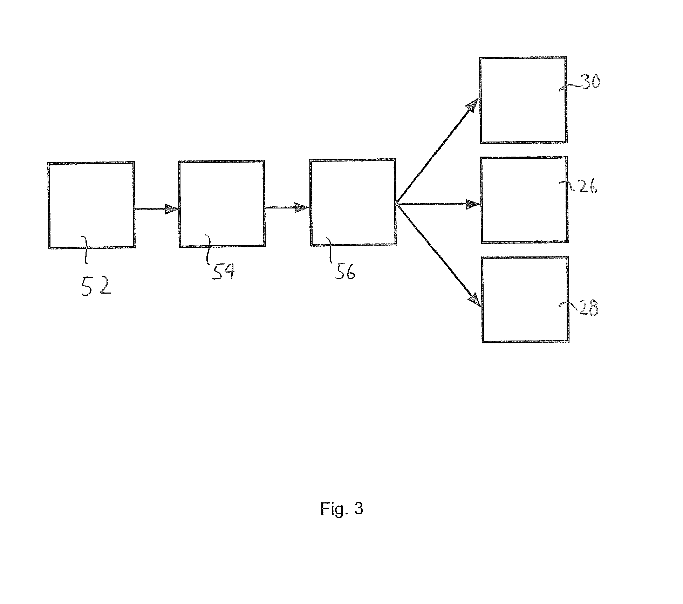

Fig. 3 is a block diagram showing the electrical components of the device 10 of Figs. 1 and 2. In Fig. 3, block 52 represents a power supply, which in the preferred embodiment may be one or more batteries, preferably rechargeable batteries.

Block 54 represents a current modulator for modulating the amplitude of the current provided by the power supply 52 with a predetermined modulation frequency. In the preferred embodiment, the modulator 54 can selectively modulate the current with a frequency of 5, 10, 1000 or 5000 Hz. However, the invention is not limited to this, and other modulation frequencies are likewise possible. However, it is advantageous if the device 10 provides at least three different modulation frequencies selected from an interval of 0.1 Hz to 10 kHz.

Block 56 represents a switching unit by which the groups of red light sources 26, blue light sources 28 and the IR light sources 30 can be selectively powered. In the present embodiment, each of the groups of light sources 26, 28 and 30 can be powered individually, and in addition, according to the switching configuration of the switching unit 56, the red light sources 26 and the IR light sources 30 can be powered together.

Next, the function of the device 10 of Figs. 1 and 2 will be described.

The device 10 of the present invention allows for a combination of different treatment modalities with what are believed to be unprecedented synergistic therapeutic effects, as described in more detail below. A particular advantage of the device 10 is that it is very flexible and versatile in its application, so that the same device can be used for different treatments, or for different stages within one treatment.

A unique feature of the device 10 is that it comprises, in both embodiments shown above, a two-dimensional array 42 of permanent magnets 32, which leads to substantial gradients in the magnetic field strength experienced by the adjacent tissue or body part. When the device 10 is moved relative to the treated body part, the direction of the magnetic flux rapidly changes in the adjacent tissue, which leads to a very effective magnetic therapy effect. This effect is further enhanced by the comparatively small gap or clearance between adjacent anti-parallel magnets 32 in the two-dimensional magnet array 42, which is essentially on a sub-millimeter scale, or even below 0.05 mm, as indicated above.

In addition to this, the device 10 of Fig. 2 allows to selectively also carry out a magnetic treatment with a single magnetic pole facing the treatment surface 18, which can be selected to be the north or south pole. It has been established in the literature that there are differences between the effect of a north pole and of a south pole facing the tissue. The north pole of a magnet causes changes in the structure of membranes leading to a relaxation and suppression of superfluous functional activity. The south pole, on the other hand, has an tonic effect and increases the functional activity of an organism, see
- Russian Patent No. 2210400, The device for electromagnetic-puncture treatments, *Ananin N.N., Klemenkov S. V., Kolesnikova I. V.,* et al.,
- Russian Patent No. 2122445, The device for magnetic therapy and light therapy, *Vilisov A.A., Antoshkin L.V., Gorlenko N.P.,* et al.,
- Solovjeva G.R., Magnetotherapeutic devices-M., Medicine.-1991.-176 P., and
- "The Device-applicator with anesthetizing magnetic stimulating and harmonizing effects 'BIOMAG"'. Registration certificate FRS 2011/10100 of February 11, 2011.

A treatment using IR and visible light is previously known in the art. An important property of red and an IR light is that the wavelengths are not absorbed by hemoglobin, so that these wavelengths allow for a better penetration in living tissue. In numerous researches it is established that IR light has anti-inflammatory, lymph-draining, vasodilating effects. The red color light restores the blood flow, metabolic processes, activates adrenals and improves immunity and potency. Blue light has antibacterial and anti-inflammatory effects.

The present invention allows for combining different effects of light therapy and magnetic therapy in a way that gives rise to new and very effective treatments, using a single device. In particular, the effect may be enhanced by combining the light and magnetic therapy with massage.

For a better illustration of the synergistic effects of the functionalities, exemplary therapeutic uses will be described in the following:

### 1. General Improvement of Health and Stimulation of Immune System

For the general improvement of health and stimulation of the immune system, the red and infrared light sources 26, 30 are switched on, and the two-dimensional array 42 of permanent magnets 32 is applied. This application is accompanied by a gentle massage (by means of the protrusions 22) that is effected when the treatment surface 18 is pressed gently against the skin and moved sideways.

This treatment can be applied to the back cervical spine, the thoracic and lumbral spine as well as on the face. When treating the face, although the light sources 26, 28 and 30 are not harmful, it is recommended to have the eyes closed.

This treatment is carried out once a day for 6-10 minutes, for 5-7 days, followed by a 2-3 month break.

The combination of red and infrared radiation and magnetic fields
- stimulates the skin and deep subcutaneous tissues,
- leads to a regeneration of muscle, connective, cartilage and bone tissue,
- increases lymph circulation and has a detoxifying effect,
- improves immune response, and
- increases body resistance.

In combination with the massage function, these effects can be increased.

### 2. Treatment for Cosmetic Purposes

In the treatment for cosmetic purposes, in a first step only the red and infrared radiation effected by the light sources 26 and 30 is employed. For this purpose, the skin on the face and the back cervical spine is irradiated without contact, keeping the treatment surface 18 2-3 cm away from the skin. Again, it is recommended to keep the eyes closed when treating the face. This procedure is preferably carried out once a day for 6-10 minutes. The treatment will be typically maintained for 5-7 days, but can be longer if necessary.

Further, this treatment may be followed by the treatment under 1.

This combination of red radiation, infrared radiation and magnetic field stimulates the skin, deep subcutaneous tissues and therefore softens fine lines, refreshes and renews tired skin. Further, it stimulates the beneficial protein-collagen synthesis. As before, it further renews muscle and connective tissues, improves immune response and it may reduce allergic reactions of the skin.

### 3. Treatment of Acne

For the treatment of acne, first, the skin is irradiated with blue light provided by the blue light sources 28 for 6-9 minutes. Here, the therapy is carried out for 2-3 days, but can be prolonged if needed. Thereafter, the skin is irradiated for 6-9 minutes once a day with a combination of red and infrared radiation provided by light sources 26 and 30, preferably for 5 to 7 days.

In both cases, the part of the skin suffering from acne is irradiated without contact but with a distance of 2-3 cm between the treatment surface 18 and the skin. If the treatment is carried out on the face, again the eyes should be closed.

This treatment is based on the observation that bacteria causing acne are sensitive to blue light. Within consecutive treatment phases with red and infrared radiation, again the immune reaction is improved, allergic reactions of the skin are reduced, and tired skin and connective tissue is refreshed and renewed.

### 4. Treatment Accompanying Fitness Training

The device 10 can further be used for people carrying out fitness training. For fitness training, again, a combination of red and infrared radiation together with magnetic field application and gentle massage can be used. This procedure can be applied one or two times a day for 5-10 minutes. Herein, the treated areas should be radiated under contact with the skin of affected muscles, tissues and joints. Bigger joints, such as shoulder, girdle and hips, should be irradiated from various sides. This is facilitated by the lightweight handheld design of the device 10.

The treatment can be used before the training, as it enables the relaxation and better preparing of muscle blood flow for strain, and decreases the risk of muscle injuries. The treatment is also beneficial after the training, as it relaxes the muscle and promotes the regeneration of muscle tissue, connective tissue and bone tissue.

A similar treatment can also be used for treating sport injuries, except that the massage should be omitted.

A further indication for the treatment with the combination of red radiation, infrared radiation and magnetic field and gentle message are neuropathies.

For the treatment of back pain, strains, dislocations and fractures, preferably red and infrared radiation and magnetic fields are used in combination, but without massage. The same is true for the treatment of arthrosis and arthritis.

The embodiments described above and the accompanying figures merely serve to illustrate the method according to the present invention, and should not be taken to indicate any limitation of the method. The scope of the patent is solely determined by the following claims.

### LIST OF REFERENCE SIGNS

- 10: handheld device
- 12: housing
- 14: handle portion
- 16: treatment portion
- 18: treatment surface
- 20: transparent cover
- 22: protrusion
- 24: carrier
- 26: red light source
- 28: blue light source
- 30: IR-light source
- 32: permanent magnet
- 34: carrier
- 36: magnet carrier
- 38: axis
- 40: arrow
- 42: array of permanent magnets
- 44: single permanent magnet
- 46: single permanent magnet
- 48: magnetic screen
- 50: handle
- 52: power supply
- 54: current modulator
- 56: switching unit

## Claims

1. A handheld combined magnetic and light therapy device (10), comprising:
- a treatment surface (18) that is to be positioned adjacent to the body part to be treated during operation;
- a two-dimensional array (42) of at least four anti-parallel magnets (32) and
- one or more light sources (26, 28, 30) arranged to emit visible and/or IR light from said treatment surface (18).

2. The device (10) of claim 1, wherein the distance between adjacent anti-parallel magnets (32) is less than 1 mm, preferably less than 0.1 mm, and most preferably less than 0.05 mm.

3. The device (10) of one of the preceding claims, wherein the two-dimensional array (42) of anti-parallel magnets (32) has a topology of a rectangular grid.

4. The device (10) of one of the preceding claims, wherein the two-dimensional array of anti-parallel magnets is provided on a movable magnet carrier (36), wherein on the movable magnet carrier, in addition to the array (42) of anti-parallel magnets (32), one or more of the following is provided:
- a single permanent magnet (44) having a north pole facing the treatment surface (18) when in operating position,
- a single magnet (46) having a south pole facing the treatment surface (18) when in operating position and
- a magnetic screen (48).

5. The device (10) of claim 4, wherein the movable carrier is rotatable, in particular around an axis (38) that is parallel to the treatment surface (18).

6. The device (10) of one of the preceding claims, wherein the one or more light sources comprises one or more of the following:
- one or more red light sources (26) having a peak wavelength in the range of 600 nm to 790 nm,
- one or more blue light sources (28) having a peak wavelength in the range of 450 nm to 480 nm, and
- one or more IR light sources (30) having a peak wavelength in the range of 790 nm to 1000 nm.

7. The device (10) of one of the preceding claims, wherein the light sources are LEDs.

8. The device (10) of one of the preceding claims, wherein the light sources are evenly distributed, and in particular circumferentially distributed around the two-dimensional array (42) of anti-parallel magnets (32).

9. The device (10) of one of the preceding claims, further comprising a plurality of protrusions (22) provided on top of the treatment surface (18), said protrusions (22) being suitable for use in a massage when the treatment surface (18) is lightly pressed against and moved relative to the treated body part.

10. The device (10) of one of the preceding claims, wherein the device comprises a transparent cover (20).

11. The device (10) of claims 9 and 10, wherein the protrusions (22) are formed on the transparent cover (20).

12. The device (10) of one of the preceding claims, further comprising a battery for powering the light sources (26, 28, 30).

13. The device (10) of one of the preceding claims, further comprising a modulating unit (54) for modulating the intensity of one or more of the light sources (26, 28, 30) with at least one frequency between 0.05 Hz and 20 kHz.

14. The device (10) of claim 13, wherein the modulation unit (54) is configured to carry out modulations at at least three different modulation frequencies selected from an interval of 0.05 Hz to 20 kHz, preferably from 0.1 Hz to 10 kHz.

15. The device of one of the preceding claims, further comprising a switching unit (56) configured to selectively switch on one or more of
- an individual light source (26, 28, 30),
- a group of plural or all of the light sources (26, 28, 30) having the same color,
- two or more groups of light sources (26, 28, 30) having the same color, and/or wherein the device (10) has a housing (12) having a handle portion (14) to be held by a user and a treatment portion (16) on which the treatment surface (18) is provided.
